(19) Europäisches Patentamt
European Patent Office
Office européen des brevets

(11) Publication number: 0 178 356 A1

(12) EUROPEAN PATENT APPLICATION

(21) Application number: 84307164.8

(22) Date of filing: 18.10.84

(51) Int. Cl.⁴: C 07 C 143/70

(43) Date of publication of application: 23.04.86 Bulletin 86/17

(84) Designated Contracting States: BE DE FR GB IT NL

(71) Applicant: SUMITOMO CHEMICAL COMPANY, LIMITED
15 Kitahama 5-chome Higashi-ku
Osaka-shi Osaka 541(JP)

(72) Inventor: Inoue, Yasuhiko
5-8, Hoshigoe-cho
Niihama-shi Ehime-ken(JP)

(72) Inventor: Hata, Kazuhiko
5-12, Hoshigoe-cho
Niihama-shi Ehime-ken(JP)

(72) Inventor: Masumoto, Katsuhisa
2-6-634, Ikku-cho
Niihama-shi Ehime-ken(JP)

(72) Inventor: Kamoda, Masaru
3-13-53, Sakai-cho
Niihama-shi Ehime-ken(JP)

(74) Representative: Moore, Anthony John et al,
Gee & Co. Chancery House Chancery Lane
London WC2A 1QU(GB)

(54) **Production of aromatic sulfonyl chlorides containing a quinonediazido group.**

(57) An aromatic sulfonic acid or its salt containing a quinonediazido group is reacted with phosgene in the prescence of a catalyst by a simplified procedure to obtain a corresponding aromatic sulfonyl chloride in high yield.

EP 0 178 356 A1

# PRODUCTION OF AROMATIC SULFONYL CHLORIDES CONTAINING A QUINONEDIAZIDO GROUP

The present invention relates to a process for producing an aromatic sulfonyl chloride containing a quinonediazido group by reacting an aromatic sulfonic acid containing a quinonediazido group or its salt with phosgene in the presence of a catalyst.

It is well known that an aromatic sulfonyl chloride having a quinonediazido group (hereinafter referred to as "quinonediazide sulfonyl chloride") is useful as an intermediate for use in preparation of organic industrial chemicals such as those used in photography, printing, dyes, and liquid crystals. With the recent marked advances in photography, printing, and electronics, quinonediazide sulfonyl chlorides have received increasing attention particularly as intermediates for producing light-sensitive agents utilizing their reactivity to light.

Heretofore, the quinonediazide sulfonyl chloride has been prepared from a quinonediazidesulfonic acid by techniques such as a method in which the quinonediazidesulfonic acid is reacted with a great excess of chlorosulfonic acid, and a method in which the quinonediazidesulfonic acid is reacted with chlorine in the presence of chlorosulfonic acid or anhydrous sulfuric acid, see for

example West German Patents 146,573 and 246,574.

These known methods, however, suffer from several disadvantages. One is that since equilibrium exists between the starting quinonediazidesulfonic acid and the reaction product (quinonediazide sulfonyl chloride), it is necessary to add chlorosulfonic acid in a great excess such as about 10 to 20 molar times theoretical if the yield of the product is to be increaded. Even so, the yield of the quinonediazide sulfonyl chloride is at most about 80%. Another disadvantage is that for removal of the unreacted starting material, a complicated procedure is required and, furthermore, a long reaction time is needed, which is undesirable. In industrial practice, therefore, these known methods cannot be said to be advantageous.

An aim of the present invention is to overcome the above defects and to provide a process for the efficient production of the quinonediazide sulfonyl chloride.

According to the present invention there is provided a process for producing a sulfonyl chloride represented by the formula (II):

$$R_i\text{-Ar-}(SO_2C\ell)_j \qquad \text{(II)}$$

by reacting a sulfonic acid or its salt represented by the formula (I):

$$R_i\text{-Ar-}(SO_3M)_j \qquad \text{(I)}$$

with phosgene in the presence of a catalyst and in the presence or absence of a solvent.

0178356

In the above formulae (I) and (II), R, which may be the same or different, is a monovalent substituent selected from a halogen atom, a nitro group, and an alkyl group having from 1 to 5 carbon atoms; Ar is an organic group of the quinonediazide structure which is substituted by at least one oxy anion and at least one diazonium cation; M is a hydrogen atom, a mono-, di- or trivalent metal, or an organic amino cation; _i_ is an integer of 0 to 5; and _j_ is an integer of 1 to 5.

Examples of the sulfonic acid or its salt of the formula (I) which can be used in the invention are aromatic sulfonic acids or their salts having a quinonediazido group, represented by the following formulae (III), (IV), and (V):

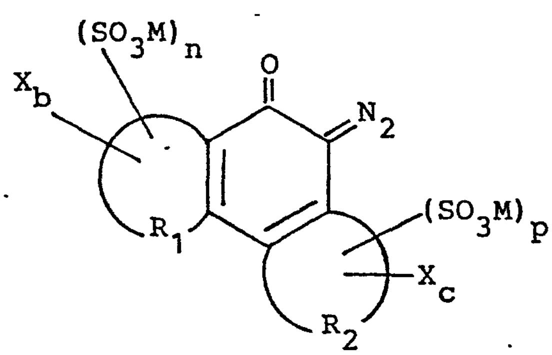

(III)

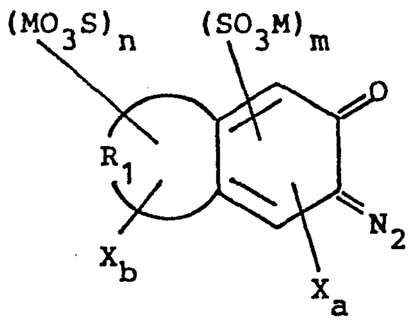

(IV)

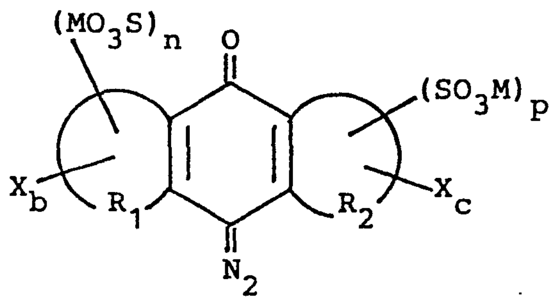

(V)

In the above formulae (III), (IV) and (V), $R_1$ and $R_2$, which may be the same or different, are each an aromatic fused ring residue represented by -C=C-C=C-, a monovalent substituent represented by $-SO_3M$, or a monovalent substituent represented by -X, in which X, which may be the same or different, is a monovalent substituent selected from a hydrogen atom, a halogen atom, a nitro group, and an alkyl group having from 1 to 3 carbon atoms; M is the same as defined above; and a, b, c, m, n and p are each 0 or a positive integer satisfying the relations that a+m=2, b+n=4, and c+p=4, provided that at least one $-SO_3M$ group is contained in the formula.

Specific examples include benzoquinonediazide-monosulfonic acid, naphthoquinonediazidemonosulfonic acid, anthraquinonediazidesulfonic acid, phenanthrene-diazidemonosulfonic acid, pyridinequinonediazidemono-sulfonic acid, quinolenequinonediazidemonosulfonic acid, chrysenquinonediazidemonosulfonic acid, benzoquinone-diazidedisulfonic acid, naphthoquinonediazidedisulfonic acid, anthraquinonediazidedisulfonic acid, phenanthrene-quinonediazidedisulfonic acid, pyridinequinonediazide-disulfonic acid, quinolenequinonediazidedisulfonic acid, chrysenquinonediazidedisulfonic acid, benzoquinonediazide-trisulfonic acid, naphthoquinonediazidetrisulfonic acid, anthraquinonediazidetrisulfonic acid, phenanthrene-quinonediazidetrisulfonic acid, pyridinequinonediazide-trisulfonic acid, quinolenequinonediazidetrisulfonic acid, chrysenquinonediazidetrisulfonic acid; their nitro, chloro, bromo or alkyl nucleus-substituted compounds; and their sodium, potassium, magnesium, calcium, barium, aluminum, trimethylammonium, triethylammonium, pyridinium, or N,N-dimethylanilinium salts.

Some more detailed examples follow:

1,4-Benzoquinonediazide-2-sulfonic acid, 1,4-benzoquinonediazide-3-sulfonic acid, 2-methyl-1,4-benzoquinonediazide-5-sulfonic acid, 2-methyl-1,4-benzoquinonediazide-6-sulfonic acid, 2-isopropyl-1,4-benzoquinonediazide-3-sulfonic acid, 2-chloro-1,4-benzoquinonediazide-5-sulfonic acid, 2-chloro-1,4-benzoquinonediazide-6-sulfonic acid, 2-bromo-1,4-benzoquinonediazide-5-sulfonic acid, 2-bromo-1,4-benzoquinonediazide-6-sulfonic acid, 2-nitro-1,4-benzoquinonediazide-5-sulfonic acid, 2,6-dimethyl-1,4-benzoquinonediazide-3-sulfonic acid, 2,6-dichloro-1,4-benzoquinonediazide-3-sulfonic acid, 2,6-dibromo-1,4-benzoquinonediazide-3-sulfonic acid, 2-chloro-6-nitro-1,4-benzoquinonediazide-3-sulfonic acid, 2-fluoro-1,4-benzoquinonediazide-5-sulfonic acid, 1,2-benzoquinonediazide-3-sulfonic acid, 1,2-benzoquinonediazide-4-sulfonic acid, 1,2-benzoquinonediazide-5-sulfonic acid, 1,2-benzoquinonediazide-6-sulfonic acid, 4-nitro-1,2-benzoquinonediazide-5-sulfonic acid, 4-chloro-1,2-benzoquinonediazide-5-sulfonic acid, 4-bromo-1,2-benzoquinonediazide-5-sulfonic acid, 6-chloro-1,2-benzoquinonediazide-4-sulfonic acid, 6-bromo-1,2-benzoquinonediazide-4-sulfonic acid, 6-chloro-1,2-benzoquinonediazide-5-sulfonic acid, 6-bromo-1,2-benzoquinonediazide-5-sulfonic acid, 6-nitro-1,2-benzoquinonediazide-5-sulfonic acid, 4-methyl-1,2-benzoquinonediazide-5-sulfonic acid, 5-methyl-1,2-benzoquinonediazide-4-sulfonic acid, 3,5-dichloro-1,2-benzoquinonediazide-4-sulfonic acid, 3,5,6-trichloro-1,2-benzoquinonediazide-4-sulfonic acid, 4-nitro-6-chloro-1,2-benzoquinonediazide-5-sulfonic acid, 1,2-benzoquinonediazide-3,5-disulfonic acid, 1,2-naphthoquinonediazide-3-sulfonic acid, 1,2-naphthoquinonediazide-4-sulfonic acid, 1,2-naphthoquinonediazide-5-sulfonic acid, 1,2-naphthoquinonediazide-6-sulfonic acid, 1,2-naphthoquinonediazide-7-sulfonic acid, 4-chloro-1,2-naphthoquinonediazide-5-sulfonic acid, 3-bromo-1,2-naphthoquinonediazide-5-sulfonic acid, 4-nitro-1,2-naphthoquinonediazide-5-sulfonic acid, 6-nitro-1,2-naphthoquinonediazide-4-sulfonic acid, 6-nitro-1,2-naphthoquinonediazide-5-sulfonic acid, 1,2-naphthoquinonediazide-3,6-disulfonic acid, 1,2-naphthoquinonediazide-4,6-disulfonic acid, 1,2-naphthoquinonediazide-4,6,8-trisulfonic acid, 2,1-naphthoquinonediazide-4-sulfonic acid, 2,1-naphthoquinonediazide-5-sulfonic acid, 2,1-naphthoquinonediazide-6-sulfonic acid, 2,1-naphthoquinonediazide-7-sulfonic acid, 2,1-naphthoquinonediazide-8-sulfonic acid, 3-chloro-2,1-naphthoquinonediazide-5-sulfonic acid, 6-chloro-2,1-naphthoquinonediazide-4-sulfonic acid, 8-chloro-2,1-naphthoquinonediazide-4-sulfonic acid, 3-bromo-2,1-naphthoquinonediazide-4-sulfonic acid, 7-bromo-2,1-naphthoquinonediazide-4-sulfonic acid, 6,8-dichloro-2,1-naphthoquinonediazide-4-sulfonic acid, 6-nitro-2,1-naphthoquinonediazide-4-sulfonic acid, 5-nitro-2,1-naphthoquinonediazide-6-sulfonic acid, 2,1-naphthoquinonediazide-3,6-disulfonic acid, 2,1-naphthoquinonediazide-4,6-disulfonic acid, 1,4-naphthoquinonediazide-5-sulfonic acid, 1,4-naphthoquinonediazide-6-sulfonic acid, 1,4-naphthoquinonediazide-7-sulfonic acid, 7-chloro-1,4-naphthoquinonediazide-7-sulfonic acid, 1,4-naphthoquinonediazide-5,7-disulfonic acid, 1,8-naphthoquinonediazide-3-sulfonic acid, 1,8-naphthoquinonediazide-6-sulfonic acid, 1,8-naphthoquinonediazide-3,6-disulfonic acid, 1,7-naphthoquinonediazide-3-sulfonic acid, 1,7-naphthoquinonediazide-3,6-disulfonic acid, 1,6-naphthoquinonediazide-3-sulfonic acid, 2,6-naphthoquinonediazide-1,4-disulfonic acid, 2-nitro-1,4-naphthoquinonediazide-7-sulfonic acid, 9,10,2,1-anthraquinonediazide-4-sulfonic acid, 9,10,2,1-anthraquinonediazide-5-sulfonic acid, 9,10,1,4-anthraquinonediazide-5-sulfonic acid, 9,10,2,1-anthraquinonediazide-5,7-disulfonic acid, 2,1-phenanthrenequinonediazide-5-sulfonic acid, 2,1-phenanthrenequinonediazide-6-sulfonic acid, 9,10-phenanthrenequinonediazide-5-sulfonic acid, 9,10-phenanthrenequinonediazide-5,7-disulfonic acid, 1-methyl-4-isopropyl-9,10-phenanthrenequinonediazide-6-sulfonic acid, 7-nitro-9,10-phenanthrenequinonediazide-5-sulfonic acid, 1-iodo-9,10-phenanthrenequinonediazide-5-sulfonic

acid, 2,3-pyridinequinonediazide-5-sulfonic acid, 4,3-pyridinequinonediazide-5-sulfonic acid, 2-methyl-4,3-pyridinequinonediazide-5-sulfonic acid, 4,3-quinolinequinonediazide-6-sulfonic acid, 4,3-quinolinequinonediazide-7-sulfonic acid, 6-chloro-4,3-quinolinequinonediazide-8-sulfonic acid, 7,8-quinolinequinonediazide-5-sulfonic acid, 1,2-chrysenquinonediazide-6-sulfonic acid, 3,4-chrysenquinonediazide-6-sulfonic acid, 8-nitro-3,4-chrysenquinonediazide-6-sulfonic acid, and 8-bromo-10-nitro-3,4-chrysenquinonediazide-6-sulfonic acid; and their sodium, potassium, magnesium, calcium, barium, aluminum, trimethylammonium, triethylammonium, pyridinium, or N,N-dimethylanilinium salts.

In particular, 1,2-benzoquinonediazide-4-sulfonic acid, 1,2-benzoquinonediazide-5-sulfonic acid, 1,2-naphthoquinonediazide-4-sulfonic acid, 1,2-naphthoquinonediazide-5-sulfonic acid, 1,2-naphthoquinonediazide-6-sulfonic acid, 1,2-naphthoquinonediazide-7-sulfonic acid, 2,1-naphthoquinonediazide-4-sulfonic acid, 2,1-naphthoquinonediazide-5-sulfonic acid, 2,1-naphthoquinonediazide-6-sulfonic acid, and 2,1-naphthoquinonediazide-7-sulfonic acid; and their sodium, potassium, calcium, or barium salts are preferably used. Especially preferred are 1,2-naphthoquinonediazide-5-sulfonic acid and 2,1-naphthoquinonediazide-4-sulfonic acid, and their sodium, potassium, calcium, or barium salts.

In accordance with the process of the present invention, the above starting material, quinonediazidesulfonic acid or its salt is reacted with phosgene to prepare the corresponding sulfonyl chloride.

In practice of the process of the present invention, as the phosgene, pure phosgene or industrial phosgene can be used.

The amount of phosgene used is usually at least 1 mole, preferably from about 1 to 2 moles, per equivalent of the sulfonic acid group present in the starting sulfonic acid or salt thereof. If the amount of phosgene used is less than 1 mole, the reaction is not completed and the starting material remains unreacted. On the other hand, if it is in excess of 2 moles, side reactions ascribable to the quinonediazide group occur, causing a reduction in yield.

Any catalysts can be used in the present invention as long as they are commonly used in sulfonyl chlorination of the sulfonic acid group. In general, acid amides, particularly lower aliphatic amides such as N,N-dialkylcarboxylic acid amides (e.g., N,N-dimethylformamide and N,N-diethylformamide), and polymers having these acid amides in the side chain are used. Among them, N,N-dimethylformamide and N,N-diethylformamide are preferably used, with N,N-dimethylformamide being particularly preferred.

The amount of the catalyst used is generally at least 0.01 mole, preferably at least 0.05 mole, and particularly preferably at least 0.2 mole, per equivalent of the sulfonic acid group present in the starting sulfonic acid or salt thereof. Since the catalyst used in the present invention can act also as a solvent, there is no special limitation with respect to the upper limit of the amount used.

The sulfonyl chlorination reaction is generally carried out in the presence of a solvent but can be performed even in the absence of a solvent. Examples of solvents which can be used include aromatic hydrocarbons such as benzene, toluene, xylene, monochlorobenzene, and dichlorobenzene; aliphatic or alicyclic hydrocarbons such as cyclohexane, hexane, n-heptane, n-octane, methylcyclohexane, 2-methylhexane, 2,3-dimethylpentane, ethylcyclohexane, cyclooctane, n-nonane, isooctane, n-decane, and n-dodecane; ethers such as diethyl ether and tetrahydrofuran; esters such as ethyl acetate and butyl acetate; acid amides such as dimethylformamide and diethylformamide; halogenated hydrocarbons such as chloroform, 1,2-dichloroethane, 1,1,2-trichloroethane, 1,1,2-trichloro-2-fluoroethane, 1,1,2-tribromoethane, 1,1,2-trichloropropane, 1,2-dichloropropane, and carbon tetrachloride; nitriles such as acetonitrile and propionitrile;

nitro compounds such as nitromethane, nitroethane, and nitrobenzene; and mineral acids such as chlorosulfonic acid, sulfuric acid, and phosphoric acid. These solvents can be used singly or in combination with each other. From viewpoints of solubility and yield of the formed sulfonyl chloride, it is preferred that lower halogenated hydrocarbons such as methylene chloride, chloroform, carbon tetrachloride, 1,2-dichloroethane, and tetrachloroethylene be used singly.

In order to increase the solubility of the reaction reagents in the solvent, it is preferred to add polar solvents such as acetonitrile, propionitrile, tetramethyl urea, dimethylformamide, diethylformamide, nitromethane, tetrahydrofuran, alkoxybenzenesulfonyl chlorides, benzenesulfonyl chloride, and phthalic chloride.

In the sulfonyl chlorination reaction, stability and reactivity vary to a certain extent depending on the type of the starting material. Also, the reactivity varies with the type and amount of the catalyst, the type of the solvent, and other reaction conditions. But the reaction temperature is usually from -30 to 100°C, preferably from -20 to 60°C.

If the reaction temperature exceeds 100°C, decomposition of the catalyst and quinonediazido group occurs, undesirably resulting in a decrease in yield.

On the other hand, if it is below -30°C, the rate of reaction is undesirably low.

The reaction can be performed under either elevated pressure or reduced pressure. Usually it is performed under around atmospheric pressure. The sulfonyl chlorination reaction of the present invention can be performed continuously, semi-continuously, or batchwise. In the case that the starting sulfonic acid or salt thereof contains water, it can be dehydrated in advance with phosgene in the presence of the catalyst, and such is rather desirable.

In the sulfonyl chlorination reaction of the present invention, it is important that phosgene be continuously supplied to a mixture prepared by mixing in advance the starting sulfonic acid or salt thereof and the catalyst and, if desired, the solvent at a rate suitable for the reactivity for sulfonyl chlorination of the starting sulfonic acid or salt thereof. If the rate of introduction of phosgene is too high as compared with the reactivity of the starting material, or if the catalyst is added in advance to a mixture of the starting sulfonic acid or salt thereof and phosgene, side reactions are caused between the starting phosgene or an intermediate formed from the starting phosgene and the catalyst and the quinonediazido group, resulting in a

decrease of yield. The reactivity for sulfonyl chlorination varies with the type of the starting sulfonic acid or salt thereof, the type and amount of the catalyst, and other conditions such as the use of the solvent and reaction temperature. Thus, taking into consideration these conditions, a suitable phosgene introduction rate may be chosen.

After the sulfonyl chlorination reaction is completed, if desired, unreacted phosgene and hydrochloric acid formed as a by-product are separated from the reaction product by techniques such as a degassing treatment (e.g., an evacuation method, a method of passing an inert gas such as nitrogen, and a combination thereof), or a water-washing treatment. The sulfonyl chlorination reaction product freed of the unreacted phosgene and by-product hydrochloric acid by the method as described above is subjected to a separation treatment such as distillation or precipitation under reduced or atmospheric pressure, to thereby remove the solvent. There is thus recovered the desired sulfonyl chloride from the sulfonyl chloride reaction product. The above precipitation treatment is achieved by introducing the reaction product into a solvent in which the sulfonyl chloride is sparingly soluble, such as ice water, benzene, and hexane. In general, there is employed a

method in which solids resulting from so-called recrystallization are filtered and separated.

The thus obtained sulfonyl chloride can be used directly or after a purification treatment such as recrystallization, in preparation of organic industrial chemicals such as those used as an intermediate for light-sensitive materials in photography or printing, dyes, or liquid crystals as well as an intermediate starting material for the preparation of photoresists.

In accordance with the process of the present invention, a sulfonyl chloride of the formula (II), $R_i$-Ar-$(SO_2C\ell)_j$ can be prepared from a sulfonic acid or its salt of the formula (I), $R_i$-Ar-$(SO_3M)_j$ and phosgene in a high yield. Further, since the reaction is completed using almost theoretical amounts of reaction reagents, it is not necessary to remove an excess of unreacted reaction reagent remaining after completion of the reaction as in conventional methods, and the reaction operation is simplified. Thus the present invention can give rise to various advantages on an industrial scale.

The present invention is described in greater detail with reference to the following Examples, although it is not intended to be limited thereby.

EXAMPLE 1

Into a mixture of 98 parts by weight of sodium 1,2-naphthoquinonediazide-5-sulfonate, 1,428 parts by weight of 1,2-dichloroethane, and 111 parts by weight of N,N-dimethylformamide was blown 43 parts by weight (1.2 times the reaction equivalent) of phosgene over 2 hours and 52 minutes with stirring while maintaining the temperature of the mixture at 0 to 25°C, to thereby achieve sulfonyl chlorination reaction. After the reaction was completed, the reaction mixture was washed four times with 271 parts by weight of ice water. The solvent, 1,2-dichloroethane, was distilled away under reduced pressure at room temperature. The residue was dried under reduced pressure at room temperature, whereupon 90 parts by weight of 1,2-naphthoquinonediazide-5-sulfonyl chloride (purity, 98.8%) was obtained. The percent yield in relation to the reaction reagent, sodium 1,2-naphthoquinonediazide-5-sulfonate, was 92%.

EXAMPLES 2 TO 20

The same procedure as in Example 1 was repeated except that the type and amount of the reaction reagent sulfonic acid or salt thereof, the solvent, and the catalyst were changed as shown in Table 1. The results are also shown in Table 1.

TABLE 1

| Example No. | Sulfonic Acid or Its Salt | | Solvent | | Catalyst | | Sulfonyl Chloride Formed | | |
|---|---|---|---|---|---|---|---|---|---|
| | Type | Amount (weight parts) | Type | Amount (weight parts) | Type | Amount (weight parts) | Yield (weight parts) | Purity (%) | Percent Yield (%) |
| 2 | Sodium 2,1-naphthoquinonediazide-4-sulfonate | 98 | Chloroform | 1,400 | N,N-Dimethylformamide | 111 | 90 | 97.8 | 91 |
| 3 | Sodium 1,2-naphthoquinonediazide-4-sulfonate | 98 | 1,1,2,2-Tetrachloroethane | 600 | N,N-Dimethylformamide | 80 | 87.6 | 98.8 | 89 |
| | | | Acetonitrile | 700 | | | | | |
| 4 | Potassium 1,2-naphthoquinonediazide-6-sulfonate | 104 | 1,2-Dichloroethane | 1,430 | N-Methyl-N-ethylformamide | 111 | 84 | 99.1 | 86 |
| 5 | Sodium 1,2-naphthoquinonediazide-7-sulfonate | 98 | Toluene | 500 | N,N-Dimethylformamide | 70 | 90 | 96.5 | 90 |
| | | | Ethyl acetate | 900 | | | | | |
| 6 | 8-Bromo-1,2-naphthoquinonediazide-5-sulfonic acid | 119 | Tetrachloroethylene | 1,200 | N,N-Dimethylformamide | 80 | 108 | 97.2 | 84 |
| 7 | 4-Nitro-1,2-naphthoquinonediazide-6-sulfonic acid | 106 | Acetonitrile | 1,480 | N,N-Dimethylformamide | 60 | 105.5 | 93.1 | 87 |

(cont'd)

| Example No. | Sulfonic Acid or Its Salt | | Solvent | | Catalyst | | Sulfonyl Chloride Formed | | |
|---|---|---|---|---|---|---|---|---|---|
| | Type | Amount (weight parts) | Type | Amount (weight parts) | Type | Amount (weight parts) | Yield (weight parts) | Purity (%) | Percent Yield (%) |
| 8 | Disodium 1,2-naphthoquinone-diazide-4,6-disulfonate | 67 | 1,2-Di-chloroethane | 1,428 | N,N-Dimethyl-formamide | 111 | 61 | 95.2 | 88 |
| 9 | Potassium 2,1-naphthoquinone-diazide-5-sulfonate | 104 | 1,1,1-Trichloro-ethane | 1,428 | N,N-Dimethyl-formamide | 111 | 91 | 96.6 | 91 |
| 10 | 2,1-Naphthoquinone-diazide-6-sulfonic acid | 90 | Xylene<br>Acetonitrile | 600<br>800 | N,N-Dimethyl-formamide | 80 | 82.6 | 98.4 | 84 |
| 11 | Dipotassium 2,1-naphthoquinone-diazide-4,6-disulfonate | 73 | Ethyl acetate<br>Cyclohexane | 700<br>700 | N,N-Dimethyl-formamide | 40 | 61 | 92.1 | 85 |
| 12 | 6-Nitro-1,2-naphtho-quinonediazide-4-sulfonic acid | 106 | Benzene<br>Acetonitrile | 600<br>800 | N-Methyl-N-ethylformamide | 80 | 100 | 96.0 | 85 |
| 13 | Sodium 5-chloro-2,1-naphthoquinone-diazide-4-sulfonate | 110 | Ethyl acetate | 1,428 | N,N-Dimethyl-formamide | 111 | 98 | 97.8 | 88 |
| 14 | 1,4-Naphthoquinone-diazide-2-sulfonic acid | 90 | Methylene chloride | 1,428 | N,N-Dimethyl-formamide | 80 | 86 | 98.2 | 87 |

(cont'd)

| Example No. | Sulfonic Acid or Its Salt | | Solvent | | Catalyst | | Sulfonyl Chloride Formed | | |
|---|---|---|---|---|---|---|---|---|---|
| | Type | Amount (weight parts) | Type | Amount (weight parts) | Type | Amount (weight parts) | Yield (weight parts) | Purity (%) | Percent Yield (%) |
| 15 | Sodium 1,2-benzoquinonediazide-4-sulfonate | 80 | 1,2-Dichloroethane | 1,428 | N,N-Dimethylformamide | 111 | 76 | 89.1 | 86 |
| 16 | 6-Methyl-1,2-benzoquinonediazide-4-sulfonic acid | 77 | 1,2-Dichloroethane | 1,428 | N,N-Dimethylformamide | 90 | 77 | 92.0 | 85 |
| 17 | Sodium 3-isopropyl-1,2-benzoquinonediazide-5-sulfonate | 95 | Chloroform | 1,428 | N-Methyl-N-ethylformamide | 60 | 92 | 86.8 | 84 |
| 18 | 3,6-Dichloro-1,2-benzoquinonediazide-4-sulfonic acid | 97 | 1,1,1-Trichloroethane | 1,200 | N,N-Dimethylformamide | 111 | 97 | 89.0 | 84 |
| 19 | Sodium 1,4-benzoquinonediazide-2-sulfonate | 80 | 1,2-Dichloroethane | 1,428 | N,N-Dimethylformamide | 111 | 73.5 | 88.9 | 83 |
| 20 | Potassium 1,4-benzoquinonediazide-3-sulfonate | 86 | Ethyl acetate | 700 | N,N-Dimethylformamide | 130 | 76.6 | 87.3 | 85 |
| | | | Acetonitrile | 700 | | | | | |

EXAMPLES 21 TO 29

The same procedure as in Example 1 was repeated except that the type and amount of the sulfonic acid or salt thereof, the amount of phosgene blown, the type and amount of the solvent, and the reaction temperature were changed as shown in Table 2. The results are also shown in Table 2.

TABLE 2

| Example No. | Sulfonic Acid or Its Salt | | Solvent | | Catalyst | |
|---|---|---|---|---|---|---|
| | Type | Amount (weight parts) | Type | Amount (weight parts) | Type | Amount (weight parts) |
| 21 | Sodium 1,2-naphthoquinonediazide-5-sulfonate | 98 | 1,2-Dichloroethane | 1,000 | N,N-Dimethylformamide | 70 |
| 22 | Sodium 1,2-naphthoquinonediazide-5-sulfonate | 98 | Chloroform | 1,200 | N,N-Dimethylformamide | 40 |
| 23 | Sodium 2,1-naphthoquinonediazide-4-sulfonate | 98 | 1,1,1-Trichloroethane | 1,400 | N,N-Dimethylformamide | 80 |
| 24 | Sodium 2,1-naphthoquinonediazide-4-sulfonate | 98 | 1,2-Dichloroethane | 1,428 | N,N-Dimethylformamide | 111 |
| 25 | Barium 1,2-benzoquinonediazide-4-sulfonate | 96 | Tetrachloroethylene | 1,500 | N,N-Dimethylformamide | 80 |
| 26 | 9,10-Anthraquinonediazide-3-sulfonic acid | 108 | 1,2-Dichloroethane | 1,500 | N,N-Methylethylformamide | 111 |
| 27 | Sodium 2,1-phenanthrenequinonediazide-5-sulfonate | 108 | 1,1,1-Trichloroethane | 1,500 | N,N-Dimethylformamide | 111 |
| 28 | Trimethylammonium 4,3-quinolinequinonediazide-8-sulfonate | 107 | 1,2-Dichloroethane | 1,500 | N,N-Dimethylformamide | 111 |
| 29 | Sodium 1,2-chrysenquinonediazide-6-sulfonate | 134 | Chloroform | 2,280 | N,N-Dimethylacetamide | 150 |

(cont'd)

| Example No. | Amount of Phosgene Blown (weight parts) | Reaction Temperature (°C) | Sulfonyl Chloride Formed | | |
|---|---|---|---|---|---|
| | | | Yield (weight parts) | Purity (%) | Percent Yield (%) |
| 21 | 38 | 35 | 89 | 96.5 | 89 |
| 22 | 54 | 0 | 85 | 97.1 | 85 |
| 23 | 38 | 55 | 85 | 94.3 | 83 |
| 24 | 54 | -10 | 86 | 98.1 | 87 |
| 25 | 48 | 20 | 69 | 98.3 | 86 |
| 26 | 48 | 20 | 101 | 95.6 | 84 |
| 27 | 43 | 20 | 102 | 97.6 | 87 |
| 28 | 43 | 10 | 88 | 94.1 | 85 |
| 29 | 43 | 20 | 119 | 92.8 | 83 |

## COMPARATIVE EXAMPLE 1

A mixture of 98 parts by weight of sodium 1,2-naphthoquinonediazide-5-sulfonate and 1,263 parts by weight of chlorosulfonic acid was stirred at a temperature of 65 to 67°C for 4 hours to achieve sulfonyl chlorination. After the reaction was completed, the reaction mixture was cooled to 20°C and poured into 8,400 parts by weight of ice water with stirring. Precipitate formed was filtered off, washed many times with water in a total amount of 12,000 parts by weight, and then dried under reduced pressure at room temperature. Thus, there was obtained 83.1 parts by weight of 1,2-naphthoquinonediazide-5-sulfonyl chloride (purity, 91.2%). The percent yield in relation to the reaction reagent, sodium 1,2-naphthoquinonediazide-5-sulfonate, was 78%.

## COMPARATIVE EXAMPLE 2

98 parts by weight of sodium 2,1-naphthoquinonediazide-4-sulfonate was mixed with 128 parts by weight of 70% fuming sulfuric acid at a temperature not more than 20°C. The mixture was heated to 50°C, and 45 parts by weight of chlorine was blown thereinto for 4 hours while stirring to achieve sulfonyl chlorination. After the reaction was completed, the reaction mixture was cooled to 20°C and then poured into 1,000 parts by

weight of ice water while stirring. Precipitate formed was filtered off, washed many times with water in a total amount of 5,000 parts by weight, and then dried under reduced pressure at room temperature. Thus, there was obtained 85 parts by weight of sodium 2,1-naphthoquinonediazide-4-sulfonate (purity, 85.8%). The percent yield in relation to the reaction reagent, sodium 2,1-naphthoquinonediazide-4-sulfonate, was 75%.

Claims:

1. A process for producing an aromatic sulfonyl chloride having a quinonediazido group, represented by the following general formula (II):

$$R_i\text{-Ar-}(SO_2C\ell)_j \qquad \text{(II)}$$

wherein R, which may be the same or different, is a monovalent substituent selected from halogen atoms, nitro groups, and alkyl groups having from 1 to 5 carbon atoms; Ar is an aromatic organic group having a quinonediazide structure which is substituted by at least one oxy anion and at least one diazonium cation; i is an integer of from 0 to 5; and j is an integer of from 1 to 5, from a sulfonic acid or a salt thereof represented by the following general formula (I):

$$R_i\text{-Ar-}(SO_3M)_j \qquad \text{(I)}$$

wherein R, Ar, i and j are as defined above; and M is a hydrogen atom, a mono-, di- or trivalent metal, or an organic amino cation, characterised in that the sulfonic acid or salt thereof represented by the formula (II) is reacted with phosgene in the presence of a catalyst and in the presence or absence of a solvent.

2. A process as claimed in Claim 1, wherein said sulfonic acid or salt thereof of the formula (I) is an aromatic sulfonic acid or a salt thereof represented by one of the following formulae (III), (IV) or (V):

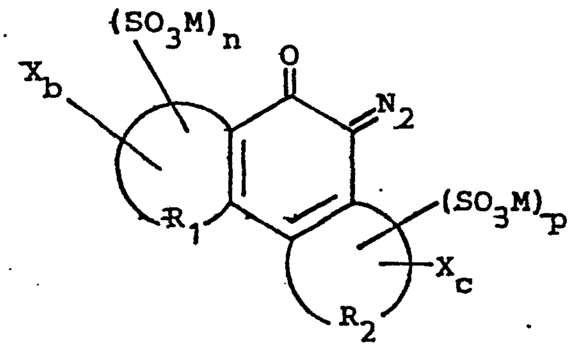

(III)

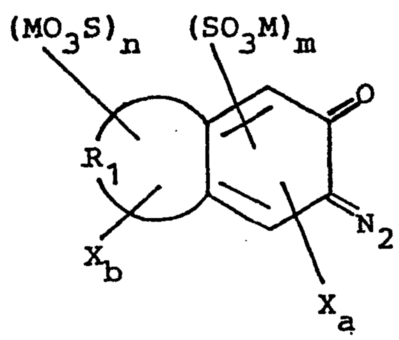

(IV)

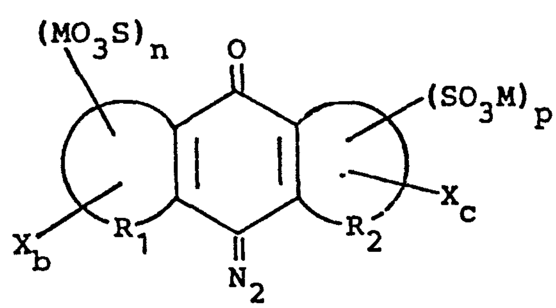

(V)

wherein $R_1$ and $R_2$, which may be the same or different, are each an aromatic fused ring residue represented by $-\overset{|}{C}=\overset{|}{C}-\overset{|}{C}=\overset{|}{C}-$, a monovalent substituent represented by $-SO_3M$, or a monovalent substituent represented by $-X$, in which X, which may be the same or different, is a monovalent substituent selected from a hydrogen atom, a halogen atom, a nitro group, and an alkyl group having from 1 to 3 carbon atoms; and M is a hydrogen atom, a mono-, di- or trivalent metal, or an organic amino cation; and $a$, $b$, $c$, $m$, $n$ and $p$ are each 0 or a positive integer satisfying the relations that $a+m=2$, $b+n=4$, and $c+p=4$, provided that at least one $-SO_3M$ group is present.

3. A process as claimed in Claim 2, wherein the aromatic ring of said aromatic sulfonic acid or salt thereof of the formula (III), (IV), or (V) is a benzene ring or a naphthalene ring.

4. A process as claimed in Claim 1, 2, or 3, wherein in the formulae (I), (III), (IV), and (V), M is a hydrogen atom, a sodium atom, a potassium atom, a magnesium atom, a calcium atom, a barium atom, an aluminum atom, a trimethylammonium group, a triethylammonium group, a pyridinium group, or an N,N-dimethylanilinium group.

5. A process as claimed in Claim 4, wherein said sulfonic acid or salt thereof (I) is 1,2-benzoquinonediazidemonosulfonic acid, 1,2-naphthoquinone-

diazideminosulfonic acid or 2,1-naphthoquinonediazidemonosulfonic acid, or a sodium, potassium, calcium or barium salt thereof.

6. A process as claimed in Claim 5, wherein said sulfonic acid or salt thereof (I) is 1,2-naphthoquinonediazide-5-sulfonic acid or 2,1-naphthoquinonediazide-4-sulfonic acid, or a sodium, potassium, calcium or barium salt thereof.

7. A process as claimed in any preceding Claim, wherein the amount of phosgene used is from 1 to 2 moles per mole of the sulfonic acid group in said sulfonic acid or salt thereof (I).

8. A process as claimed in any preceding Claim, wherein said catalyst is an acid amide.

9. A process as claimed in Claim 8, wherein said acid amide is N,N-dimethylformamide.

10. A process as claimed in any preceding Claim, wherein the amount of catalyst used is at least 0.01 mole per mole of the sulfonic acid group in said sulfonic acid or salt thereof(I).

11. A process as claimed in any preceding Claim, wherein said solvent contains methylene chloride, chloroform, carbon tetrachloride, 1,2-dichloroethane or tetrachloroethylene.

12. A process as claimed in any preceding Claim, wherein the reaction temperature is from -30°C to 100°C.

European Patent Office

# EUROPEAN SEARCH REPORT

0178356
Application number

EP 84 30 7164

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| Y | CHEMICAL ABSTRACTS, vol. 96, no. 5, 1st February 1982, page 621, no. 34766b, Columbus, Ohio, US; B.Z.YAKOVLEV et al.: "Preparation of o-naphthoquinonediazide sulfonyl chlorides" & KHIM. PROM-ST. (MOSCOW) 1981, (8), 505<br>--- | 1 | C 07 C 143/70 |
| Y | CHEMICAL ABSTRACTS, vol. 100, no. 13, 26th March 1984, page 611, no. 102940g, Columbus, Ohio, US; & JP - A - 58 183 665 (SUMITOMO CHEMICAL CO., LTD.) 26-10-1983<br>--- | 1,8-10 | |
| Y | EP-A-0 001 277 (BAYER)<br>* Claims *<br>----- | 1 | TECHNICAL FIELDS SEARCHED (Int. Cl.4)<br>C 07 C 143/00 |

The present search report has been drawn up for all claims

| Place of search | Date of completion of the search | Examiner |
|---|---|---|
| THE HAGUE | 06-06-1985 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document